# EUROPÄISCHE PATENTANMELDUNG

(11) **EP 2 606 862 A1**
(43) Veröffentlichungstag der Anmeldung: **26.06.2013**
(21) Anmeldenummer: 12198930.5
(22) Anmeldetag: 21.12.2012
(51) Int. Cl.: A61F 7/12

(54) **Medizinische Vorrichtung zum endovaskulären Kühlen und/oder Erwärmen von Blut**

(30) Priorität: 23.12.2011 DE 102011057009
(71) Anmelder: Acandis GmbH & Co. KG, 76327 Pfinztal (DE)
(72) Erfinder: Cattaneo, Giorgio, 76199 Karlsruhe (DE)
(74) Vertreter: Kilchert, Jochen

(57) **Zusammenfassung**

Die Erfindung bezieht sich auf eine medizinische Vorrichtung zum endovaskulären Kühlen oder Erwärmen von Blut mit einem komprimierbaren und expandierbaren Wärmetauscher (10), der in ein Blutgefäß einführbar ist und wenigstens einen Kanal (11) zum Durchströmen mit einem Kühl- oder Heizmedium aufweist, wobei der Kanal (11) durch wenigstens eine Kanalwand (12) begrenzt ist, die im Gebrauch mit dem Blut in Berührung kommt und für den Wärmeübergang zwischen dem Medium und dem Blut angepasst ist. Sie zeichnet sich dadurch aus, dass der Wärmetauscher (10) eine selbstexpandierbare Stützstruktur (13) mit einer Wandung (14) bildet, die im expandierten Zustand ein für das Blut durchströmbares Lumen begrenzt und eine radiale Rückstellkraft erzeugt, die im Gebrauch als Stützkraft auf das Blutgefäß wirkt, wobei der Kanal zumindest abschnittsweise in die Wandung (14) integriert und/oder mit der Wandung (14) fest verbunden ist.

## Beschreibung

Erfindung betrifft eine medizinische Vorrichtung zum endovaskulären Kühlen und/oder Erwärmen von Blut mit den Merkmalen des Oberbegriffs des Anspruchs 1. Eine medizinische Vorrichtung dieser Art ist beispielsweise aus US 6,702,783 B1 bekannt.

Eine häufige Komplikation bei der Rekanalisation eines Blutgefäßes nach einem ischämischen Schlaganfall ist die Blutung. Dies hängt damit zusammen, dass die nekrotischen Bereiche distal vom Gefäßverschluss Gefäße mit degenerierter Gefäßwand aufweisen. Wenn es bei der Rekanalisation zu einem plötzlichen Blutdurchfluss kommt, der mit der Wiederherstellung des arteriellen Blutdruckes einhergeht, kann es zur Beschädigung distaler Gefäße und Blutungen kommen. Dieses Problem tritt besonders bei mechanischen Rekanalisationssystemen auf, die meist zu einer schnellen Rekanalisation des betroffenen Gefäßes führen. Demgegenüber weist die medikamentöse Behandlung einer Thrombose sowohl venös als auch arteriell das Problem der verstärkten Neigung zur Blutung auf. Ferner bekannt sind die positiven Effekte der Hypothermie, die sich beispielsweise in einer Reduzierung des Stoffwechsels (langsames Absterben von Zellen), in der Hemmung von Entzündungsprozessen sowie in der Reduzierung von Hämatomen bei Blutungen äußern. Gerade bei der Behandlung von Schlaganfällen hat die Hypothermie entscheidende Vorteile und kann zur Verlängerung des Zeitfensters bei der Behandlung sowie zur Reduzierung von Nebenwirkungen beitragen. Im Gegensatz zur Lyse ist die Hypothermie auch zur Behandlung von hämorrhagischen Schlaganfällen geeignet. Die Hypothermie kann deshalb als eine der ersten Behandlungsmaßnahmen unabhängig von der Art des Schlaganfalls (ischämisch oder hämorrhagisch) eingesetzt werden. Dazu werden Patienten mit Schlaganfall von außen abgekühlt bspw. durch Kühlwesten oder Kühlhauben, wodurch große Blutvolumina betroffen sind. Die extrakorporale Abkühlung des Blutes hat den Nachteil, dass diese zu einer Kühlung des Herzens führt. Dadurch ist die Abkühltemperatur begrenzt, was wiederum nicht ausreichen könnte, um eine wirksame Hypothermie-Behandlung durchzuführen. Überdies kommt es zu unerwünschten Nebenwirkungen bei anderen Organen.

Die lokale Abkühlung, beispielsweise des Gehirns, kann dadurch erreicht werden, dass das Blut aus der Carotis entnommen, extrakorporal abgekühlt und in die Carotis durch eine Blutpumpe wieder zurückgeführt wird. Die extrakorporale Blutzirkulation hat verschiedene Nachteile. Es kann zur Hämolyse, also zum Zerfall roter Blutkörperchen kommen, weil das Blut in dünnen und langen Lumen gefördert wird. Es besteht ein beträchtliches Infektionsrisiko sowie das erhöhte Risiko der Bildung von Thromben aufgrund der großen Kontaktfläche. Außerdem sind extrakorporale Kreislaufunterstützungssysteme sehr aufwändig und in der Praxis nur in der Kardiochirurgie und auf Intensivstationen einsetzbar.

Eine entscheidende Verbesserung wird durch die endovaskuläre Hypothermie erzielt, bei der der Wärmeübergang vom Blut auf ein anderes Medium zum Abkühlen des Blutes direkt im Gefäß erfolgt. Die endovaskuläre Hypothermie bringt den Vorteil, dass einerseits eine sehr lokale Kühlwirkung erzeugt wird. Andererseits entfallen alle mit der extrakorporalen Blutabkühlung verbundenen Probleme. Für die endovaskuläre Hypothermie werden Ballonkatheter verwendet, wie sie beispielsweise in der US 6,702,783 B1 beschrieben sind, die mittels eines Katheters in das zu behandelnde Blutgefäß eingeführt werden. Der Ballon dient dabei als Wärmetauscher, der mit einem Kühlmittel versorgt wird. Das Kühlmittel dient gleichzeitig dazu, den Ballon im Gefäß zu expandieren. Zum Entfernen des Ballons wird dieser in an sich bekannter Weise deflatiert und kann in das Zufuhrsystem wieder eingezogen werden.

Der bekannte Ballonkatheter weist im Bereich des Wärmetauschers verdrillte Schläuche auf, die sich im Gebrauch im Lumen des Blutgefäßes erstrecken. Daraus ergibt sich ein erheblicher Strömungswiderstand, der zumindest bei einer längerfristigen Behandlung die Blutversorgung distal gelegener Gefäße behindert. Der Ballonkatheter ist außerdem nur zur Behandlung großer Gefäße geeignet. Gerade im zerebralen Bereich ist der Ballonkatheter nicht einsetzbar, da die Gefäße in diesem Bereich für den Ballonkatheter zu klein sind und die Gefahr der Gefäßverletzung besteht.

Aufgabe der Erfindung ist es daher, eine medizinische Vorrichtung zum endovaskulären Kühlen und/oder Erwärmen von Blut der eingangs genannten Art anzugeben, die im Gebrauch einen verbesserten Blutfluss ermöglicht und miniaturisierbar ist derart, dass sich die Vorrichtung insbesondere zur Behandlung kleiner Gefäße, wie beispielsweise zerebraler Gefäße, eignet.

Diese Aufgabe wird durch eine medizinische Vorrichtung mit den Merkmalen des Anspruchs 1 gelöst. Insbesondere wird die Aufgabe durch eine medizinische Vorrichtung zum endovaskulären Kühlen oder Erwärmen von Blut mit einem komprimierbaren und expandierbaren Wärmetauscher gelöst, der in ein Blutgefäß einführbar ist und wenigstens ein Führungsmittel aufweist. Das Lumen des Führungsmittels ist mit einem Kühl- oder Heizmedium durchströmbar und weist eine Wand auf, deren Innenseite zumindest teilweise das Lumen des Führungsmittels begrenzt. Die Außenseite der Wand kommt im Gebrauch zumindest teilweise mit dem Blut in Berührung. Die Wand ist für den Wärmeübergang zwischen dem Medium und dem Blut angepasst. Der Wärmetauscher weist eine selbstexpandierbare Wandung auf, die im expandierten Zustand die Außenkontur des Wärmetauschers bildet. Das Führungsmittel bildet zumindest abschnittsweise die Wandung oder ist zumindest abschnittsweise mit der Wandung verbunden derart, dass das Führungsmittel beim Expandieren der Wandung mitbewegt wird. Generell kann für das Führungsmittel Kunststoff, Metall oder ein anderes wärmeleitendes Material verwendet werden. Nitinol ist ebenfalls möglich.

Mit der Erfindung werden folgende Vorteile erreicht.

Die Ausbildung der Wandung als selbstexpandierbare Wandung bedeutet, dass für die Expansion keine äußeren Kräfte erforderlich sind, wie bei einem Ballon. Die Rückstellkräfte sind innere Kräfte, die bspw. durch die Ausbildung der Wandung durch ein Formgedächtnismaterial, wie Nitinol oder andere SMA-Materialien, erzielt werden. Formelastische Materialien, die durch die Komprimierung eine Federkraft erzeugen, sind beispielsweise auch möglich. Die selbstexpandierbare Wandung des Wärmetauschers führt zu einer einfachen Herstellung und Handhabung der Vorrichtung. Der einfache Aufbau ermöglicht überdies die Miniaturisierung der Vorrichtung. Hierfür kann auf die im Zusammenhang mit bekannten Rekanalisationssystemen entwickelten komprimierbaren Systeme zurückgegriffen werden, die für den Einsatz in sehr kleinen Gefäßen, insbesondere im zerebralen Bereich, hervorragend geeignet sind.

Durch die zumindest abschnittsweise Integration des Führungsmittels in die selbstexpandierbare Wandung bzw. durch die Ausbildung der selbstexpandierbaren Wandung durch das Führungsmittel selbst wird die Voraussetzung dafür geschaffen, dass das Führungsmittel und damit die Wärmetauscherfläche bzw. Wärmetauscherflächen gezielt im Gefäß positioniert werden können, um Toträume zu vermeiden, in denen das Blut nicht oder zumindest weniger stark gekühlt wird. Außerdem erlaubt die selbstexpandierbare Wandung die Positionierung des Führungsmittels mit einer radialen Komponente, die quer oder schräg zur Strömungsrichtung im Gefäß verläuft. Die Länge und der Anstellwinkel des in das Lumen des Gefäßes hineinragenden Führungsmittels sind durch die Geometrie der Wandung einfach einstellbar und beeinflussen den Wärmeübergang durch Konvektion oder Konduktion. Dies wird bspw. dadurch erreicht, dass die Wandung als Gitterstruktur ausgebildet ist, die einen Teil oder den gesamten Strömungsquerschnitt überdeckt. Je größer der von der Wandung überdeckte Anteil des Strömungsquerschnittes ist, umso größer sind die Turbulenzen, die den Wärmeübergang verbessern. Wenn der Strömungswiderstand verringert werden soll, kann das Führungsmittel aus dem Strömungsquerschnitt des Blutgefäßes weitgehend oder sogar vollständig entfernt wird. Da die Wandung die Außenkontur des expandierten Wärmetauschers bildet, wird dieser zumindest auf einer Seite von Blut angeströmt und leistet den gewünschten Wärmeübergang. Besser noch ist der Wärmetauscher im Gebrauch von der Gefäßwand beabstandet angeordnet, so dass die Wandung von außen und von innen mit Blut anströmbar ist. Der Strömungswiderstand ist gering, weil das Führungsmittel in die Wandung integriert ist bzw. die Wandung bildet bzw. mit der Wandung verbunden ist. Damit folgt das Führungsmittel der Form der Wandung, die strömungstechnisch günstig ausgebildet sein kann.

Die Integration des Führungsmittels in die Wandung bzw. die feste Verbindung des Führungsmittels mit der Wandung schließt nicht aus, dass das Führungsmittel zumindest teilweise in das Lumen des Gefäßes hineinragt. Da das Führungsmittel an der die Außenkontur bildenden Wandung vorgesehen ist, ragt dieses von radial außen nach radial innen in das Gefäßlumen hinein. Damit ist die Tiefe, mit der das Führungsmittel in das Lumen hineinragt, einstellbar. Dies ist bei den bekannten Ballonkathetern nicht möglich. Überdies kann das Führungsmittel ausgehend von der Wandung auch radial nach außen oder radial nach innen und außen vorstehen.

Zwar ist aus EP 1 525 013 A1 eine medizinische Vorrichtung bekannt, die spiralförmige Gasleitungen aufweist. Die Gasleitungen sind in einer Hülle bzw. in einem Gehäuse angeordnet, das allerdings nicht mit den Gasleitungen verbunden ist. Die Hülle wird vielmehr durch den Blutdruck radial ausgedehnt und übt somit keine Stützwirkung auf das Gefäß aus. Abgesehen davon, dass es sich bei dieser Vorrichtung um einen Oxygenator zur Zuführung von Sauerstoff zum Blut handelt, sind die Gasleitungen jedenfalls nicht mit der Hülle bzw. dem Gehäuse fest verbunden. Dies ist auch nicht möglich, da dann die Axialbewegung auf den mit den Gasleitungen verbunden Schlitten entlang der Katheterleitung blockiert wäre.

Weitere Ausgestaltungen der Erfindung sind in den Unteransprüchen angegeben.

Bei einer bevorzugten Ausführungsform bildet das Führungsmittel wenigstens eine flexible Hohlfaser. Diese ist einfach mit der Wandung verbindbar. Im Unterschied zu den durch das Kühlmittel im Radius expandierbaren Schläuchen der Ballonkatheter ist die Hohlfaser im wesentliche durchmesserstabil. Die Formänderung erfolgt unabhängig vom Druck des Mediums in der Hohlfaser. Die Wand der Hohlfaser ist stabil gegen den Druck des Mediums. Die Formänderung der Hohlfaser folgt der Formänderung der selbstexpandierbaren Wandung. Wenn die durchmesserstabile Hohlfaser selbst die Wandung bildet, ist die Formänderung der Hohlfaser für die Formänderung der Wandung ursächlich. Die gewünschte Formänderung, um in den Expansionszustand zu gelangen, ist der Hohlfaser aufgeprägt, bspw. auf Grund des Formgedächtniseffektes.

Bei einer besonders bevorzugten Ausgestaltung weist die Wandung eine Gitterstruktur auf, insbesondere eine geflochtene oder lasergeschnittene Gitterstruktur. Die Gitterstruktur bietet die Möglichkeit eine ausreichend große Rückstellkraft der selbstexpandierbaren Wandung zu erzeugen, um das Führungsmittel beim Expandieren mit der Wandung mitzubewegen und so eine präzise einstellbare Positionierung der Wärmetauscherflächen im Gefäß zu erreichen. Die Expansionsmechanismen von Gitterstrukturen sind bekannt und gut beherrschbar. Außerdem lässt sich die Gitterstruktur besonders gut miniaturisieren und ist damit zur Behandlung kleiner Gefäße besonders geeignet.

Die Ausbildung des Führungsmittels als Hohlfaser hat den Vorteil, dass deren Außenform bzw. Außenkontur im Wesentlichen der Form der Gitterelemente der Gitterstruktur entspricht. Die Hohlfaser ist daher gut in die Gitterstruktur integrierbar bzw. gut mit der Gitterstruktur verbindbar. Dabei kann das Führungsmittel eine einzige oder mehrere Hohlfasern umfassen. Die Gitterstruktur kann geflochten sein oder lasergeschnitten sein. In beiden Fällen bildet die Ausbildung des Führungsmittels als Hohlfaser die Möglichkeit, dieses so mit der Gitterstruktur zu kombinieren, dass das Führungsmittel der Gitterstruktur bei der Expansionsbewegung folgt.

Die Hohlfaser kann teilweise oder vollständig die geflochtene Gitterstruktur der Wandung bilden. Damit ersetzt die Hohlfaser teilweise oder vollständig die Gitterelemente der Gitterstruktur, die aus Vollmaterial hergestellt sind, bspw. Gitterdrähte. Dies hat den Vorteil, dass die gute Positionierbarkeit der Wandung zu einer ebenso guten Positionierbarkeit des Führungsmittels führt, so dass das Blut möglichst homogen gekühlrt werden kann.

Die Hohlfaser bzw. die Hohlfasern können in die Gitterstruktur eingeflochten oder mit der Gitterstruktur stoffschlüssig verbunden sein. Im ersten Fall sind die Hohlfasern in die Gitterstruktur integriert, wobei die Hohlfasern auch zusätzlich mit der Gitterstruktur fest verbunden sein können. Im zweiten Fall sind die Hohlfasern mit der Gitterstruktur fest verbunden bspw. an einer Innenseite und/oder Außenseite der Gitterstruktur. Die stoffschlüssige Verbindung kann beispielsweise durch Kleben oder Schweißen, insbesondere Laserschweißen, erfolgen. Andere Verbindungsarten zwischen der Gitterstruktur und den Hohlfasern sind möglich, wie mechanische Verbindungen, bspw durch Crimpen oder durch lokales Verflechten der tragenden Gitterstruktur mit der temperierenden Gitterstruktur, die nachfolgend beschrieben ist. Die Hohlfasern können eine weitere Gitterstruktur, d.h. eine temperierende Gitterstruktur bilden, die mit der tragenden Gitterstruktur der Wandung fest verbunden ist. Dadurch wird eine flächige Ausbildung des Wärmetauschers erreicht, wodurch eine besonders große Wärmetauscherfläche zur Verfügung gestellt wird.

Die Wandung des Führungsmittels kann fluiddicht oder porös sein. Im ersteren Fall findet kein Stoffaustausch zwischen dem Kühl- oder Heizmedium und dem Blut statt. Die Abkühlung bzw. Erwärmung des Blutes erfolgt ausschließlich durch den Wärmeübergang durch die Wand. In diesem Fall ist die Wand besonders dünn. Alternativ kann die Wand der Hohlfasern porös sein, so dass das Medium durch die Wand diffundieren kann. In diesem Fall ist eine physiologische Flüssigkeit zu verwenden, wie beispielsweise eine Kochsalzlösung. Der Wärmeübergang erfolgt zusätzlich durch den Massetransport.

Zur Erhöhung der Wirksamkeit des Wärmetauschers kann dieser wenigstens eine Zwischenlage aufweisen, die sich in Richtung der Blutströmung erstreckt und wenigstens ein Führungsmittel für das Kühl- oder Heizmedium aufweist. Das Führungsmittel kann zumindest abschnittsweise in die Zwischenlage integriert oder fest mit dieser verbunden sein. Auch hier gilt, dass das Führungsmittel zusätzlich zur Integration in die Zwischenlage mit dieser fest verbunden sein kann. Die Zwischenlage vergrößert die wirksame Wärmetauscherfläche, ohne dabei den Strömungswiderstand im Lumen des Gefäßes spürbar zu erhöhen, da die Zwischenlage sich in der Richtung der Blutströmung erstreckt und somit seitlich von Blut umströmt wird. Damit werden nicht temperierbare Toträume vermieden oder verkleinert.

Die Wandung kann bei einer bevorzugten Ausführungsform wenigstens zwei gliederartige Einzelsegmente aufweisen, die nachfolgend angeordnet und unabhängig voneinander komprimierbar und expandierbar sind. Diese Ausgestaltung hat den Vorteil, dass die Vorrichtung sehr flexibel ist und somit in stark gewundene Gefäße eingeführt werden kann. Außerdem wird das Foreshortening begrenzt, da die Einzelsegmente unabhängig voneinander komprimierbar bzw. expandierbar sind, so dass insgesamt eine vergleichsweise geringe Längenänderung mit der Durchmesseränderung einhergeht. Der besondere Vorteil liegt darin, dass die Wandung der Einzelsegmente und damit die der Wandung zugeordneten Führungsmittel präzise positionierbar sind. Insbesondere kann die Ausbildung der radialen Komponente der Wandung in Strömungsrichtung mehrfach hintereinander erfolgen (serielle Anordnung), wodurch die Wirksamkeit verbessert wird.

Das Führungsmittel kann für die Zufuhr des Mediums mit einem Einlass und für die Abfuhr des Mediums mit einem Auslass jeweils zur Verbindung mit einer Versorgungseinrichtung für das Medium verbunden sein. Die Zufuhr und Abfuhr ermöglichen eine kontinuierliche Versorgung des Wärmetauschers mit entsprechend temperiertem Kühl- bzw. Heizmedium. Dadurch ist eine besonders wirkungsvolle Hypothermie-Behandlung möglich. Der Wärmetauscher kann bspw. wenigstens ein Führungsmittel für die Zufuhr des Mediums und wenigstens ein weiteres Führungsmittel für die Abfuhr des Mediums aufweisen, wobei die beide Führungsmittel miteinander fluidverbunden sind und einen Einlass bzw. einen Auslass zur Verbindung mit einer Versorgungseinrichtung für das Medium aufweisen. Es ist auch möglich, das Führungsmittel in der Form einer Hohlfaser vorzusehen, deren freien Enden an ein und demselben Axialende, insbesondere am proximalen oder distalen Ende, oder an verschiedenen Axialenden des Wärmetauschers vorgesehen und mit der Versorgungseinrichtung verbunden oder verbindbar sind.

Alternativ kann der Wärmetauscher ein Reservoir aufweisen, das mit dem Führungsmittel verbunden und im Gebrauch mit dem Medium füllbar bzw. entleerbar ist. Diese Ausgestaltung ermöglicht eine pulsative Versorgung des Wärmetauschers und ist konstruktiv besonders einfach herstellbar. Für die Versorgung ist eine einzige Leitung ausreichend.

Bevorzugt sind mehrere Führungsmittel in gesonderten Modulen angeordnet, deren Fläche jeweils kleiner als die Fläche der gesamten Wandung des Wärmetauschers und/oder die Länge der Module jeweils kürzer als die Gesamtlänge der Wandung bzw. des Wärmetauschers ist. Damit lassen sich gezielt lokale Gefäßbereiche kühlen.

Um den Druckabfall in dem Führungsmittel zu verringern, sind die Module unabhängig voneinander mit dem Medium versorgbar oder strömungstechnisch parallel geschaltet. Die Parallelschaltung erfolgt dadurch, dass eine gemeinsame Zuleitung wenigstens zwei Module versorgt, die sich stromabwärts vor den Modulen in zwei Teilleitungen aufteilt, die jeweils mit einem Modul verbunden sind.

Gemäß einem nebengeordneten Aspekt beruht die Erfindung auf dem Gedanken, ein medizinisches System bzw. ein Set mit der zuvor erläuterten Vorrichtung und einer Zufuhreinrichtung, insbesondere einer Schleuse, anzugeben. Die medizinische Vorrichtung kann längsverschieblich in der Schleuse angeordnet sein. Die Schleuse ist vorzugweise mit einem Katheter zur Einfuhr der medizinischen Vorrichtung in ein Körperhohlgefäß, insbesondere ein Blutgefäß, verbindbar.

Die Erfindung wird nachfolgend anhand von Ausführungsbeispielen weiter erläutert und beschrieben, die in den beigefügten schematischen Zeichnungen dargestellt sind. Dabei zeigen
- Fig. 1: eine Seitenansicht einer Vorrichtung für die endovaskuläre Hypothermie mit einer Gitterstruktur nach einem erfindungsgemäßen Ausführungsbeispiel;
- Fig. 2: in stark schematisierter Weise einen Querschnitt durch eine Vorrichtung für die endovaskuläre Hypothermie nach einem weiteren Ausführungsbeispiel, nach dem die Führungsmittel teilweise in das Lumen ragen;
- Fig. 3: einen Längsschnitt durch eine Vorrichtung für die endovaskuläre Hypothermie nach einem weiteren Ausführungsbeispiel mit mehreren Zwischenschichten;
- Fig. 4: einen Längsschnitt durch eine Vorrichtung für die endovaskuläre Hypothermie mit mehreren Einzelsegmenten;
- Fig. 5: einen Längsschnitt durch eine Vorrichtung für die endovaskuläre Hypothermie nach einem weiteren Ausführungsbeispiel mit im expandierten Zustand gewölbter Wandung; und
- Fig. 6: einen Längsschnitt durch eine Vorrichtung für die endovaskuläre Hypothermie nach einem weiteren Ausführungsbeispiel mit einer weiteren Versorgungsleitung.

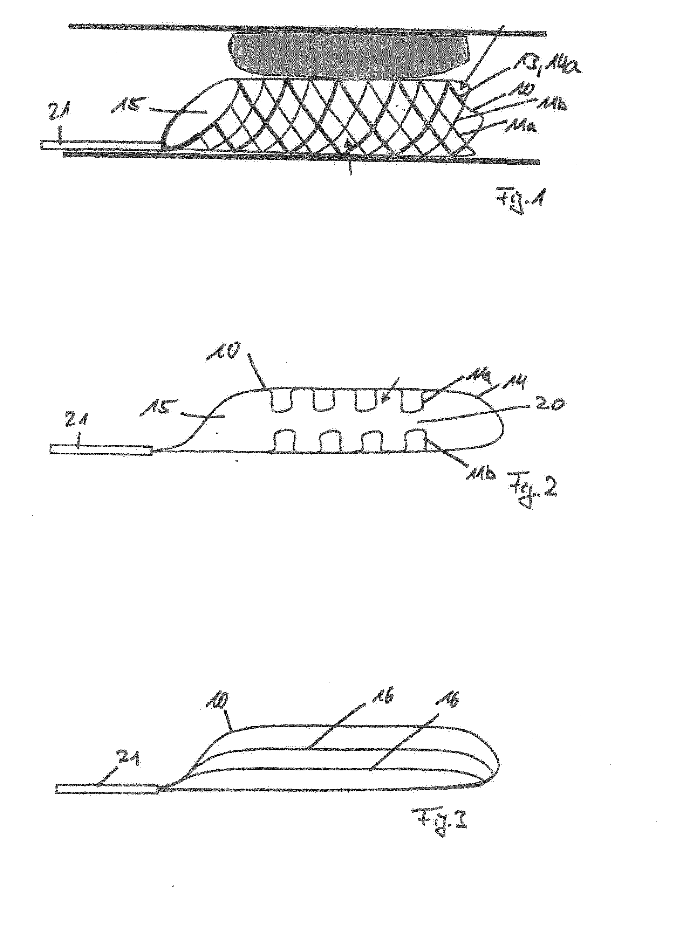

Fig. 1 zeigt in einer Seitenansicht eine medizinische Vorrichtung zum endovaskulären Kühlen von Blut (endovaskuläre Hypothermie) im Gebrauch. Die Vorrichtung kann auch zum endovaskulären Erwärmen von Blut (endovaskuläre Hyperthermie) verwendet werden, indem anstatt eines Kühlmediums ein Heizmedium durch das Führungsmittel 11 geleitet wird. Dies gilt für alle Ausführungsbeispiele in dieser Anmeldung.

Die Vorrichtung ist in an sich bekannter Weise durch ein Zufuhrsystem 21, wie beispielsweise einen Katheter, im Bereich des Verschlusses platziert. Eine andere Anordnung im Gefäß ist möglich. Dazu umfasst die Vorrichtung einen komprimierbaren und expandierbaren Wärmetauscher 10, der in der Form eines Stents oder Rekanalisierungsdevices ausgebildet ist. Zum Aufbau der Grundstruktur des Stents wird auf die deutsche Anmeldung 10 2009 056 450 verwiesen, die auf die Anmelderin zurückgeht. Konkret ist der Wärmetauscher 10 mit einer selbstexpandierbare Wandung ausgebildet, die ohne äußere Krafteinwirkung nach dem Entlassen aus dem Zufuhrsystem expandiert. Durch die Expansion bildet die Wandung 14 ein für Blut durchströmbares Lumen 15 aus. Konkret ist die Wandung 14 röhrchenförmig ausgebildet. Andere Geometrien bspw. wellenförmige Profile mit einer radialen Wandungskomponente oder ein konisches Profil oder ein Profil mit einem variablen Durchmesser, z.B. proximal kleiner Durchmesser, mittig größerer Durchmesser und distal wieder kleiner Durchmesser (im Unterschied zu einer zylindrischen Mantelfläche) sind möglich. Im Gebrauch kann die sich aus der Selbstexpandierbarkeit ergebende radiale Rückstellkraft zu einer Stützkraft führen, die, wie in Fig. 1 zu sehen, auf das Gefäß wirkt und dieses aufweitet. Dies ist aber nicht zwingend.

Durch eine geeignete Dimensionierung kann der Wärmetauscher 10 auch so im Gefäß platziert werden, dass die Wandung 14 von der Gefäßwand zumindest bereichsweise beabstandet ist und keine Stützfunktion übernimmt. Der Wärmetauscher wird dann von allen Seiten umströmt, wodurch sich die wirksame Wärmetauscherfläche vergrößert.

Die Wandung 14 weist eine selbstexpandierbare Gitterstruktur 15 auf. Die Mechanismen, die bei einer selbstexpandierbaren Gitterstruktur zur Expansion führen, sind an sich bekannt und werden hier nicht näher beschrieben. Die Gitterstruktur besteht teilweise aus massiven Gitterelementen und teilweise aus flexiblen Hohlfasern 11a, 11b. Der Durchmesser und/oder die Querschnittsform der Hohlfasern 11a, 11b kann vom Durchmesser bzw. der Querschnittsform der massiven Gitterelemente abweichen oder diesen entsprechen. Der Durchmesser der Hohlfasern 11a, 11b kann größer als der Durchmesser der massiven Gitterelemente sein. Es ist auch möglich, die gesamte Gitterstruktur aus Hohlfasern auszubilden. Die Hohlfasern sind an sich bekannt. Die Hohlfasern 11a, 11b bilden Leitungen, die in die Wandung 13 integriert sind. Das bedeutet, dass die Hohlfasern 11a, 11b Teil der Wandung 14 sind und sich zumindest abschnittsweise oder vollständig in der Wandungsebene befinden. In Fig. 1 sind die massiven Gitterelemente, d.h. die selbstexpandierbaren Unterstützungsdrähte der Gitterstruktur 15, fett gedruckt. Die dünnen Linien in Fig. 1 stellen die Hohlfasern 11a, 11b dar, die Teil der Gitterstruktur 15 sind. Die Hohlfasern 11a, 11b können zur Bildung eines Gittergeflechts miteinander und/oder mit den selbstexpandierbaren Unterstützungsdrähten verflochten sein. Zu den möglichen Flechtmustern wird auf die vorgenannte deutsche Anmeldung 10 2009 056 450 beispielhaft verwiesen. Aufgrund des in Fig. 1 dargestellten schrägen proximalen Umfangsrandes ist die Gitterstruktur 15 einfach in das Zufuhrsystem 21 wiedereinziehbar.

Die Hohlfasern 11a, 11b sind mit einer Versorgungseinrichtung für das Medium verbunden, wobei ein Teil der Hohlfasern 11a als Zufuhrmittel dient, das einen mit der Versorgungseinrichtung verbundenen Einlass aufweist (nicht dargestellt). Der andere Teil der Hohlfasern 11b ist als Abflussmittel ausgebildet, das einen Auslass aufweist, der ebenfalls mit der Versorgungseinrichtung für das Medium verbunden ist (nicht dargestellt). Damit ist ein kontinuierliches Durchströmen des Wärmetauschers 10 mit Kühlmedium möglich.

Die Versorgungsleitungen für die ersten und zweiten Führungsmittel 11a, 11b für die Zu- bzw. Abfuhr des Mediums sind im Zufuhrsystem 21 angeordnet und am proximalen Ende des Zufuhrsystems mit der Versorgungseinrichtung verbunden. Die Versorgungseinrichtung weist in an sich bekannter Weise eine Kühl- bzw. allgemein Temperiereinrichtung für das Medium sowie eine Pumpe für den Transport des Mediums zum bzw. vom Wärmetauscher 10 auf.

Bei dem Ausführungsbeispiel gemäß Fig. 1 bilden die Hohlfasern 11a, 11b einen Teil der tragenden Struktur der Wandung 14 und können somit zur Erzeugung der radialen Rückstellkraft beitragen. Dadurch wird ein besonders platzsparender Wärmetauscher bereitgestellt, der in kleinen Gefäßen einsetzbar ist.

Die Führungsmittel 11a, 11b können auch als Mikrokapillaren oder generell als Leitungen oder Röhrchen bezeichnet werden. Die Hohlfasern 11a, 11b können einen Außendurchmesser aufweisen, der kleiner als 250 µm, insbesondere kleiner als 200 µm, insbesondere kleiner als 150 µm, insbesondere kleiner als 100 µm, insbesondere kleiner als 50 µm, insbesondere als 25 µm ist. Diese Werte bilden jeweils Obergrenzen. Die Untergrenze kann 23 µm betragen. Die Wandungsstärke kann kleiner als 100 µm, insbesondere kleiner als 50 µm, insbesondere kleiner als 25 µm, insbesondere kleiner als 20 µm, insbesondere kleiner als 15 µm, insbesondere als 10 µm sein. Diese Werte bilden jeweils Obergrenzen. Die Untergrenze kann 8 µm betragen. Derartige Strukturen sind bspw. durch an sich bekannte Dünnschichttechnologien, wie Sputtern herstellbar, die auf röhrchenförmige Strukturen anwendbar sind. Dies gilt für alle Ausführungsbeispiele dieser Anmeldung.

Alternativ zu der vorbeschriebenen Integration der Führungsmittel 11a, 11b in die Wandung 14 durch Verweben oder Verflechten, können die Hohlfasern 11a, 11b eine gesonderte Struktur bilden, die beispielsweise als weiteres Gittergeflecht ausgebildet ist. Dadurch wird eine flächige Ausbildung des Wärmetauschers 10 erreicht. Die weitere Gitterstruktur kann mit der Gitterstruktur 15 der Wandung beispielsweise verklebt oder verschweißt sein. Vorzugsweise ist die weitere Gitterstruktur aus den Hohlfasern 11a, 11b auf der Innenseite und/oder der Außenseite der Gitterstruktur angeordnet. Die weitere Gitterstruktur aus Hohlfasern 11a, 11b kann auch im Zusammenhang mit einem lasergeschnittenen Stent verwendet werden, wobei die Wandung 14 aus Stegen besteht, an denen die weitere Gitterstruktur aus Hohlfasern 11a, 11b befestigt ist. Auch hier bietet es sich an, die Gitterstruktur aus Hohlfasern 11a, 11b auf der Innenseite und/oder der Außenseite der Wandung 14 zu befestigen.

Vorzugsweise weist der Wärmetauscher 10 mindestens 5, insbesondere mindestens 10, insbesondere mindestens 20, insbesondere mindestens 50, insbesondere mindestens 100 mal so viele Hohlfasern 11a, 11b als Drähte bzw. bzw. als massive Gitterelemente auf.

Bei dem Ausführungsbeispiel gemäß Fig. 1 befinden sich die Hohlfasern 11a, 11b im Wesentlichen in derselben Wandungsebene wie die massiven Gitterelemente der Stützstruktur. Alternativ können, wie in Fig. 2 dargestellt, die Hohlfasern 11a, 11b zumindest teilweise in das Lumen des Wärmetauschers 10 hineinragen. Die Hohlfasern 11a, 11b bilden dabei ein inneres Lumen 20, durch das das Blut frei strömen kann. Da die Hohlfasern 11a, 11b an der Innenseite der Wandung 14 befestigt sind bzw. in diese integriert sind, kann der Durchmesser des inneren Lumens 20 beliebig variiert bzw. vergrößert werden, um den Strömungswiderstand zu verändern. Die aus Fig. 2 ersichtliche radiale Komponente der Hohlfasern 11, 11b kann durch eine Geometrie erreicht werden, die sich von der Geometrie der Wandung unterscheidet. Bspw. Ist es möglich die Hohlaern 11a, 11b als separates Geflecht mit einem anderen Flwechtwinkel als die Gitterstruktur 15 auszubilden auf Grund der sich daraus ergebenden unterschiedlichen Längenänderungen beim Expandieren, wird die Wellenform erreicht, ggf. unterstützt durch entsprechende lokale Befestigungsstellen des Geflechts an der Gitterstruktur.

Eine weitere Möglichkeit der Ausführung des Wärmetauschers 10 ist in Fig. 3 dargestellt. Zur Vergrößerung der Wärmetauscherflächen sind mehrere Zwischenlagen 16 vorgesehen, die sich in Richtung der Blutströmung, also in längsaxialer Richtung der Vorrichtung erstrecken. Dadurch wird der Strömungswiderstand nur unwesentlich erhöht. Die Zwischenlagen sind wie die äußere Wandung 14 ausgebildet und weisen beispielsweise eine Gitterstruktur auf, in die die Hohlfasern 11a, 11b integriert bzw. mit der die Hohlfasern 11a, 11 fest verbunden sind. Hierzu wird auf die Ausführungen zu Fig. 1 verwiesen. Die Lage kann jeweils wie in Fig. 2 mit aus der Wandungsebene vorstehenden Führungsmitteln ausgebildet sein. Auf die Erläuterungen zu Fig. 2 wird Bezug genommen.

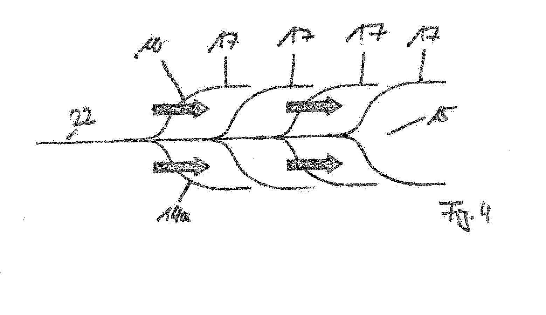

Wenn der Wärmeübergang verbessert werden soll, kann die in Fig. 4 dargestellte Form der Stützstruktur 10 verwendet werden, bei der mehrere Einzelsegmente 17 nachfolgend in längsaxialer Richtung der Vorrichtung, d.h. in Richtung der Blutströmung angeordnet sind. Die Einzelsegmente 17 sind jeweils mit einer Wandung aus einer Gitterstruktur versehen und damit durchströmbar. Die Einzelsegmente weisen, wie in Fig. 4 dargestellt, einen distalen zylindrischen Abschnitt auf, der sich in proximaler Richtung verjüngt und konusartig zusammenläuft. Das proximale Ende des jeweiligen Einzelsegments 17 ist mit einem Führungsdraht bzw. Transportdraht 22 fest verbunden (nur schematisch dargestellt). Die Zuleitung kann durch den hohlen Transportdraht oder durch eine separate Leitung (nicht dargestellt) erfolgen. Durch die Anordnung des konusartigen proximalen Endes der Einzelsegmente 17 im Strömungsquerschnitt des Blutgefäßes werden turbulente Strömungsverhältnisse begünstigt, wodurch der Wärmeaustausch durch Konvektion verbessert wird. Aufgrund der Gitterstruktur der Einzelsegmente 17 wird der Strömungswiderstand nur unwesentlich erhöht. Die Vorrichtung gemäß Fig. 4 hat den weiteren Vorteil, dass diese aufgrund der Einzelsegmente 17 insgesamt sehr flexibel ist und sich daher besonders gut zur Behandlung von kleinen und stark gewundenen Gefäßen eignet.

Im Zusammenhang mit den gitterförmigen Tragstrukturen bzw. Wandungen wird offenbart, dass die Hohlfasern porös sein können. Bei porösen Hohlfasern ist der Durchfluss der Flüssigkeiten bzw. das Diffundieren der Flüssigkeiten (Blut und Kühlmittel) durch die Wandung der Hohlfasern 11a, 11b möglich. Wenn für die Kühlflüssigkeit eine physiologische Lösung verwendet wird, ist die unproblematisch. Die Verwendung von mikroporösen Hohlfasern 11a, 11b hat den Vorteil, dass die Abgabe des Kühlmediums gesteuert und homogen in einem gewissen Volumen zu gewährleisten ist. Die Abkühlung durch Konduktionsprozesse, d.h. durch den Temperaturunterschied zwischen der Innen- und Außenfläche der Faserwand, bleibt weiterhin vorhanden. Die Verwendung mikroporöser Fasern führt nicht zwangsläufig zu einem Durchfluss durch die Wandung der Faser, weil Oberflächenspannungen verhindern, dass Flüssigkeit vom Innenlumen in das Außenlumen dringt. Die Durchlässigkeit der Hohlfasern 11a, 11b kann durch die Dimension der Poren und/oder den Druck des Kühlmediums in den Hohlfasern eingestellt werden. Die Hohlfasern bzw. Kapillaren können auch dicht sein. Dann wird die Wärme nur durch Konduktion übertragen, wobei in diesem Fall die Faserwand sehr dünn und das Material entsprechend wärmeleitend ist. Die vorgenannten Ausbildungen der Wände werden explizit für alle Beispiele offenbart, insbesondere für das Beispiel gemäß Fig. 2. Die Hohlfasern können beispielsweise metallisch, beispielsweise aus Nitinol sein. In diesem Fall bilden die Hohlfasern 11a, 11b selbst die tragende Struktur.

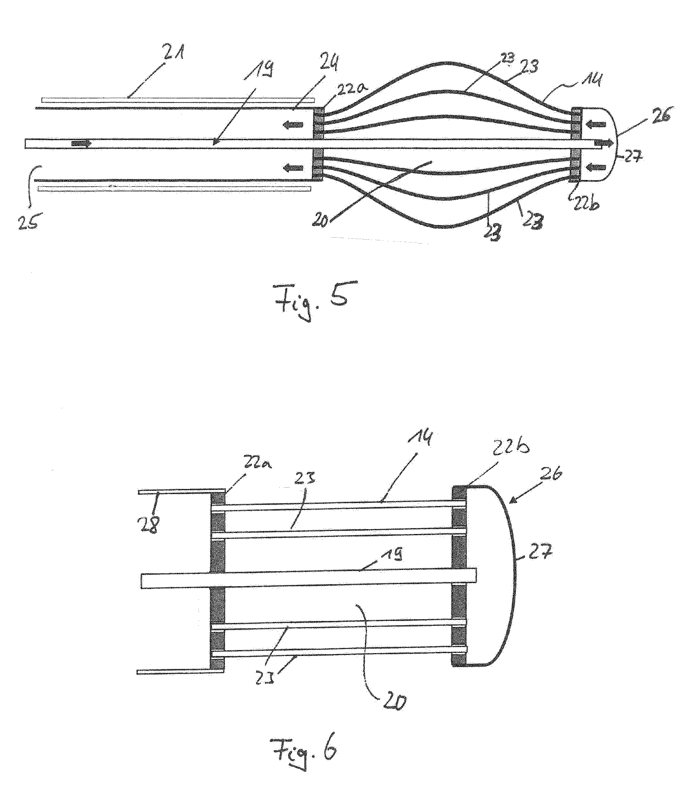

Ein weiteres Beispiel für eine selbsttragende Struktur des Wärmetauschers 10 bzw. Wärmeübertragers ist in Figur 5 dargestellt. Bei dem Beispiel gemäß Figur 5 bildet der Wärmetauscher 10 einen Expansionskörper, der aus mehreren flexiblen Röhrchen, beispielsweise Metallröhrchen 23 gebildet ist. Die Röhrchen 23 bilden Einzelröhrchen. Die Metallröhrchen 23 können beispielsweise aus einem Formgedächtniswerkstoff, wie beispielsweise Nitinol, gebildet sein. Die Röhrchen bilden im expandierten Zustand die in Figur 5 gezeigte gekrümmte Form mit einem maximalen Abstand zwischen den Röhrchen 23 in etwa in der Mitte des Wärmetauschers 10. Andere Formen des Expansionselements bzw. der Röhrchen 23, die das Expansionselement bilden, sind möglich.

Der Wärmetauscher 10 ist selbstexpandierbar. Dazu befinden sich die Röhrchen 23 im gestreckten Zustand im Zufuhrsystem 21 bzw. im Katheter, wodurch eine Expansion der Röhrchen 23 in radialer Richtung verhindert wird. Wenn der Wärmetauscher 10 aus dem Zufuhrsystem 21 entlassen wird, expandieren die Röhrchen 23 in radialer Richtung des Wärmetauschers 10. Die Röhrchen 23 gehen dabei von der gestreckten, im wesentlichen geraden Form oder von einer gewellten Form (komprimierter Zustand) in eine gekrümmte, insbesondere kontinuierlich gekrümmte Form über (expandierter Zustand). Im expandierten Zustand bilden die Röhrchen 23 die in Fig. 5 gezeigte gewölbte Wandung 14.

Im Beispiel gemäß Fig. 5 sind 6 Röhrchen 23 in der dargestellten Schnittebene vorgesehen. Eine andere Anzahl von Röhrchen ist möglich.

Die Röhrchen 23 sind von radial außen nach radial innen nebeneinander angeordnet. Die weiter außen angeordnet Röhrchen 23 sind stärker gekrümmt als die radial weiter innen angeordneten Röhrchen 23. Es ist grundsätzlich auch möglich, dass alle Röhrchen 23 eine gleiche oder zumindest sehr ähnliche Krümmung aufweisen. So ist sichergestellt, dass sich der Wärmetauscher zur Anordnung in einer Zufuhreinrichtung 21, insbesondere in einem Katheter oder in einer Schleuse, gleichmäßig bzw. homogen verformt, da sich jedes Röhrchen 23 bei der Komprimierung um die gleiche Länge verlängert bzw. streckt.

Die Röhrchen 23 bilden verschiedene Lagen, die in unterschiedlichen Abständen zur Versorgungsleitung 19 angeordnet sind. Weitere Röhrchen 23 sind auf dem Umfang des Wärmetauschers 10 verteilt angeordnet. Mehrere Röhrchen 23 einer Lage sind in derselben Umfangsfläche angeordnet. Die Röhrchen 23 können eine einzige Lage bilden.

Die Röhrchen 23 sind symmetrisch zur Versorgungsleitung 19 angeordnet. Mindestens ein Röhrchen 23, konkret 3 Röhrchen ist/sind jeweils beidseitig von der Versorgungsleitung 19 angeordnet. Eine andere Anzahl von Röhrchen 23 ist möglich.

Für die Fluidversorgung weist der Wärmetauscher 10 gemäß Fig. 5 wenigstens eine Versorgungsleitung 19 auf, die einen ersten Strömungskanal für das Temperierfluid bildet. Der Wärmetauscher 10 ist mit der Versorgungsleitung 19 fluidverbunden. Der Wärmetauscher 10 weist ferner einen Dichtungsabschnitt 24 auf, der mit dem Wärmetauscher 10 verbunden und zur Abdichtung gegen das Zufuhrsystem 21 angepasst ist. Der Dichtungsabschnitt 24 bildet wenigstens einen zweiten Strömungskanal, der mit dem Wärmetauscher 10 fluidverbunden ist und eine proximale Mündungsöffnung 25 aufweist. Die Mündungsöffnung 25 ist distal vom proximalen Ende der Versorgungsleitung 19 angeordnet.

Die Versorgungsleitung 19 ist im Lumen des Dichtungsabschnitts 24 und im Lumen des Zufuhrsystems 21 angeordnet. Das Zufuhrsystem 21 kann eine Katheterleitung sein. Dies gilt für alle Beispiele.

Vorzugsweise, aber nicht zwingend, dichtet der Dichtungsabschnitt 24 gegen die Versorgungsleitung 19 ab.

Der Dichtungsabschnitt 24 ist als schlauchförmiges Element ausgebildet. Der Durchmesser des Dichtungsabschnitts 24 ist etwas kleiner als der Innendurchmesser des Zufuhrsystems 10, so dass der Dichtungsabschnitt 24 gut im Zufuhrsystem 10 gleitet und zugleich die Dichtfunktion erfüllt, wenn der Wärmetauscher 10 freigesetzt wird.

Der Dichtungsabschnitt 24 bewirkt, dass die Fluidversorgung des Wärmeübertragers 10 einerseits durch die Versorgungsleitung 19 und andererseits durch die Zufuhreinrichtung bzw. das Zufuhrsystem 21 erfolgen kann, so dass die Versorgung durch zwei getrennte Fluidströme, bspw. für die Fluidzufuhr und die Fluidabfuhr möglich ist. Damit kann der Wärmetauscher 10 kontinuierlich betrieben werden, was für eine konstante Temperierleistung günstig ist. Außerdem entfällt im proximalen Bereich des Wärmeübertragers 10 die im Stand der Technik übliche Doppelleitung, bei der zwei Versorgungsleitungen nebeneinandergeführt und mit dem Wärmeübertrager 10 verbunden sind. Der Außendurchmesser der gesamten Leitungsanordnung Wärmeübertragers 10 wird dadurch verringert, der somit bspw. in kleinlumigen Gefäßen einsetzbar ist. Die Temperierleistung, insbesondere Kühlleistung wird dabei, bezogen auf einen von den Außendimensionen vergleichbaren Wärmeübertrager, verbessert, da der Strömungsquerschnitt durch die Verringerung der Anzahl der Leitungen vergrößert wird.

Die getrennte Führung der Fluidströme, zumindest proximal vom Wärmeübertrager 10, wird durch die Versorgungsleitung 19 einerseits und durch den Dichtungsabschnitt 24 andererseits bewirkt, der mit dem Wärmeübertrager 10 verbunden ist. Im Gebrauch strömt durch die Versorgungsleitung 19 ein erster Fluidstrom kontrolliert, d.h. von der Umgebung abgegrenzt, in den oder aus dem Wärmeübertrager 10. Insofern bildet die Versorgungsleitung 19 einen ersten Strömungskanal.

Der Dichtungsabschnitt 24 bildet einen zweiten Strömungskanal, der mit dem Wärmeübertrager 10 fluidverbunden ist. Der erste und zweite Strömungskanal sind voneinander fluidgetrennt, so dass im zweiten Strömungskanal das Fluid getrennt vom Fluid im ersten Strömungskanal und abgetrennt von der Umgebung strömen kann. Das Fluid kann im ersten und zweiten Strömungskanal bspw. im Gegenstrom strömen.

Die proximale Mündungsöffnung 25 des zweiten Strömungskanals ist distal vom proximalen Ende der Versorgungsleitung 19 angeordnet. Im einfachsten Fall ist bspw. der zweite Strömungskanal axial kürzer als der erste Strömungskanal. Dadurch wird erreicht, dass der Fluideintritt und Fluidaustritt im Leitungssystem an unterschiedlichen Stellen erfolgen. Durch die unterschiedlichen Ein- und Austrittsstellen des Fluids wird die Doppelfunktion des Zufuhrsystems einerseits zur Fluidversorgung des Temperierelements und andererseits für die mechanische Zufuhr des Wärmeübertragers 10 ermöglicht. Die Versorgungsleitung 19 kann dabei in an sich üblicher Weise an eine Fluid- und Temperiereinrichtung angeschlossen werden, insbesondere extrakorporal angeschlossen werden. Der zweite Strömungskanal des Dichtungsabschnitts 24 steht im Gebrauch mit dem Zufuhrsystem 21 in Fluidverbindung, das als eine Verlängerung des zweiten Strömungskanals fungiert. Das Zufuhrsystem 21 kann den zweiten Strömungskanal bspw. mit einem extrakorporalen Anschluss oder einem proximal beanstandeten hämostatischen Ventil verbinden.

Unter Mündungsöffnung 25 wird dabei eine freie, d.h. nicht unmittelbar mit einer Leitung verbundene Öffnung verstanden, durch die im Gebrauch Fluid in das Innere oder aus dem Inneren des Strömungskanals strömt. Bspw. ist die Mündungsöffnung 25 am axialen Ende des Strömungskanals angeordnet.

Die von der Versorgungsleitung 19 getrennte Fluidverbindung zwischen dem zweiten Strömungskanal und der Zufuhreinrichtung 21 wird dadurch ermöglicht, dass die proximale Mündungsöffnung 25 des zweiten Strömungskanals distal vom proximalen Ende der Versorgungsleitung 19 angeordnet ist. Im einfachsten Fall genügt es deshalb, dieselbe Leitung der Zufuhreinrichtung 21, durch die der Wärmeübertrager 10 mechanisch zugeführt wird, mit der Fluidversorgung oder einem Fluidausgang, wie einem hämostatischen Ventil zu verbinden, um die Fluidverbindung mit dem zweiten Strömungskanal herzustellen.

Die Fluidverbindung zwischen dem Dichtungsabschnitt 24 und der Zufuhreinrichtung 21 wird dadurch verbessert, dass der Dichtungsabschnitt 24 zur Abdichtung gegen die Zufuhreinrichtung 21 angepasst ist. Bspw. kann eine Außenfläche des Dichtungsabschnitts 24 als Dichtfläche ausgebildet sein, die mit einer Innenwand der Zufuhreinrichtung, insbesondere der Innenwand einer Transportleitung der Zufuhreinrichtung 21 bzw. dem Zufuhrsystem 21 im Gebrauch dichtend zusammenwirkt.

Dabei ist es nicht erforderlich, dass der Dichtungsabschnitt 24 vollständig verhindert, dass Temperierfluid in die Blutbahn gelangt. Es genügt, wenn die in das Blut gelangende Menge an Temperierflüssigkeit so weit verringert wird, dass das Blut nicht verwässert wird. Konkret kann eine ausreichende Dichtwirkung dann angenommen werden, wenn weniger als 15%, insbesondere weniger als 10%, insbesondere weniger als 5%, insbesondere weniger als 2% des Temperierfluids in die Blutbahn gelangen. Da das Temperierfluid üblicherweise eine physiologische Lösung ist, ist das Entweichen von Temperierfluid aus der Vorrichtung unbedenklich, solange keine Verwässerung des Blutes erfolgt. Es ist möglich, dass sich der Dichtungsabschnitt 24 bis zum proximalen Ende des Zufuhrsystems 21 (nicht dargestellt) erstreckt. So wird ein Flüssigkeitsübergang vom Dichtungsabschnitt 24 in das Zufuhrsystem 21 vermieden. Eine Abdichtung durch den Dichtungsabschnitt 24 ist dann nicht erforderlich. Vielmehr kann der Dichtungsabschnitt 24 in diesem Fall ein Betätigungselement bilden, so dass die Vorrichtung durch den Dichtungsabschnitt 24 durch das Zufuhrsystem 21 bzw. die Zuführeinrichtung 24 verschoben wird.

Für die Fluidversorgung der Röhrchen 23 sind die Enden der Röhrchen 23 im Bereich des distalen Endes des Wärmetauschers 10 mit einem Verteiler 26 fluidverbunden. Der Verteiler 26 ist mit dem distalen Ende der Versorgungsleitung 19 fluidverbunden, so dass aus der Versorgungsleitung 19 Temperierfluid über den Verteiler 26 in die Röhrchen 23 strömen kann. Eine Umkehr der Strömungsrichtung ist möglich. Die proximalen Enden der Röhrchen 23 sind mit einem proximalen Anschlussstück 22a verbunden. Das proximale Anschlussstück 22a ist mit dem distalen Ende des Dichtungsabschnitts 24 verbunden und bildet jeweils Durchführungen für die einzelnen Röhrchen 23, so dass insgesamt eine Fluidverbindung zwischen den Röhrchen 23, dem Dichtungsabschnitt 24 und dem Zufuhrsystem 21 besteht.

Grundsätzlich gilt für alle Ausführungsformen der Erfindung, dass die Vorrichtung mit einer Versorgungsleitung 19 und/oder einem Dichtungsabschnitt 24 versehen sein kann, wobei inbesondere die Versorgungsleitung 19 oder der Dichtungsabschnitt 24 gleichzeitig ein Betätigungselement bilden können.

Eine weitere, insbesondere zentrale, Durchführung im proximalen Anschlussstück 22a ist für die Versorgungsleitung 19 vorgesehen. Die Versorgungsleitung 19 ist mit dem proximalen Anschlussstück 22a zur Kraftübertragung auf den Wärmetauscher 10 verbunden. Die Versorgungsleitung dient somit als Betätigungselement, um den Wärmetauscher 10 im Zufuhrsystem 21 zu bewegen. Alternativ ist die Versorgungsleitung 19 lose im proximalen Anschlussstück 22a angeordnet, so dass eine Relativbewegung möglich ist. Die Versorgungsleitung 19 erstreckt sich durch das Lumen 20 des Wärmetauschers 10 und mündet in den Verteiler 26.

Der Verteiler 26 ist durch ein distales Anschlusstück 22b mit den Röhrchen 21 und der Versorgungsleitung 19 verbunden. Die beiden Anschlussstücke 22a, 22b sind entsprechend aufgebaut. Eine Kappe 27 ist mit dem distalen Anschlussstück 22b verbunden und bildet einen Verteilerraum, in den die Röhrchen 23 und die Versorgungsleitung 19 münden.

Wenn die Versorgungsleitung 19 mit dem distalen Anschlusselement 22b fest verbunden ist und im proximalen Anschlussstück 22a gleitet, verkürzt sich die Vorrichtung beim Entlassen durch die Wölbung der Wandung 14 nach außen. Eine solche Vorrichtung hat den Vorteil des einfachen Aufbaus. Die Röhrchen 23 sind im komprimierten Zustand gerade und gleiten gut im Zufuhrsystem 21 bzw. in der Katheterleitung.

Alternativ ist die Versorgungsleitung 19 mit dem proximalen Anschlusselement 22a und mit dem distalen Anschlusselement 22b jeweils fest verbunden. Wenn die Versorgungsleitung zugfest ist, wird eine Verkürzung der Vorrichtung vermieden. Dadurch wird die Auswölbung der Wandung 14 nach außen beschränkt und ist dementsprechend voreinstellbar. Die Röhrchen 23 wellen sich im komprimierten Zustand im Katheter.

Eine Alternative zu dem vorstehend genannten Beispiel besteht darin, dass die Versorgungsleitung 19 mindestens zwischen beiden Verbindungsstellen, d.h. zwischen dem proximalen und distalen Anschlussstück 22a, 22b elastisch dehnbar ist und sich bei der Streckung der Vorrichtung verformt. Die Versorgungsleitung 19 besteht zB. aus einem Geflecht oder einer Gitterstruktur, die mit einem Kunststoff (bspw. PU) ummantelt wird, oder aus einem reinen Kunststoff. Eine derart ausgebildete Versorgungsleitung 19 kann mit dem proximalen und distalen Anschlussstücken 22a, 22b fest verbunden sein, wobei eine Streckung der Vorrichtung im Zufuhrsystem einschließlich der damit verbundenen Verlängerung der Vorrichtung vermieden wird.

Die Röhrchen 23 können aus Nitinol gebildet sein.

Die Röhrchen 23 können spiralförmig angeordnet sein. Bspw. können die Röhrchen 23 zwischen den beiden Anschlussstücken 22a, 22b mindestens eine ¼ Umdrehung, insbesondere mindestens eine ½ Umdrehung, insbesondere mindestens eine ganze Umdrehung vollziehen.

Die Versorgungsleitung 19 kann im Bereich der Anschlussstücke 22a, 22b einen Durchmesser von mindestens 0,2 - 0,4 des gesamten Durchmesser der Anschlussstücke 22a, 22b und von höchstens 0,8 - 0,6 des gesamten Durchmesser der Anschlussstücke 22a, 22b aufweisen.

Die Versorgungsleitung 19 kann einen Außendurchmesser zwischen 0,4 mm und 0,1 mm, insbesondere zwischen 0,3 mm und 0,2 mm und eine Wandung mit einer Dicke kleiner als 60 µm, insbesondere kleiner als 50 µm, insbesondere kleiner als 40 µm aufweisen. Die Anzahl der Röhrchen 23 kann mindestens 5, insbesondere mindestens 10, insbesondere mindestens 20, insbesondere mindestens 30, insbesondere mindestens 40 betragen. Die maximale Anzahl ergibt sich aus der Komprimierbarkeit und der Strömungsdurchlässigkeit der Vorrichtung und wird vom Fachmann ermittelt. Die Länge der Röhrchen 23 beträgt zwischen 10 mm und 100 mm, insbesondere zwischen 10 mm und 60mm, insbesondere zwischen 30 mm und 60 mm. Das Verhältnis zwischen der Summe der Querschnitte aller Röhrchen 23 und dem gesamten Querschnitt des proximalen und/oder distalen Anschlussstücks 22a, 22b kann mindestens 40%, insbesondere mindestens 50%, insbesondere mindestens 60%, insbesondere mindestens 70%, betragen.

Zu den Geometriemerkmalen der Röhrchen 23 und des zugehörigen Katheters (Zufuhrsystem 21) wird auf die folgende Tabelle verwiesen:

| Außendurchmesser Röhrchen | Wandung | Anzahl | Innendurchmesser Katheter |
|---|---|---|---|
| | | | |
| 0,3 - 0,4 mm | ≤ 60 µm | > 5, 8, 10 | ∼ 2mm |
| | | | |
| 0,2 - 0,3 mm | ≤ 50 µm | > 30, 40 | ∼ 2mm |
| | | > 5, 10, 15 | ∼1 mm |
| | | | |
| 0,1 - 0,2 mm | ≤ 40 µm | > 5, 10, 15, 20 | 0,7mm |
| | | | |

In der Praxis funktioniert der Wärmetauscher 10 gemäß Fig. 5 wie folgt: Der Wärmetauscher 10 wird in der Zufuhreinrichtung zur Behandlungsstelle transportiert und im Bereich der Behandlungsstelle, bspw. proximal zu einer Lesion, platziert. Dort temperiert, insbesondere kühlt, dieser das vorbeiströmende Blut. Der Dichtungsabschnitt 24 stellt dabei die Fluidverbindung zwischen dem Wärmeübertrager 10 und einer Fluidversorgung oder einem Fluidausgang zusätzlich zur Versorgungsleitung 19 her, so dass ein kontinuierlicher Betrieb des Temperierelements möglich ist. Eine Gegenstromführung des Temperierfluids in der Versorgungsleitung 19 und im Dichtungsabschnitt 24 ist bevorzugt. Bspw. fungiert die Versorgungsleitung 19 als Zufuhrleitung, wobei das Temperierfluid in der Versorgungsleitung 19 zum Wärmeübertrager 10 hinströmt. Der Dichtungsabschnitt 24 und die Zufuhreinrichtung 21 wirken als Abfuhr- oder Rücklaufleitung, wobei das Temperierfluid vom Wärmeübertrager 10 wegströmt. Eine Umkehrung der Strömungsrichtungen ist möglich.

Der kompakte Aufbau der Vorrichtung macht diese besonders für die Anwendung miniaturisierter Temperierelemente geeignet, die in kleinen, distalen Gefäßen platziert werden und eine gute Kühlleistung aufweisen. Eine mögliche Anwendung ist die Kühlung cerebraler Gefäße. Vorzugsweise wird die Vorrichtung zum Kühlen von Blut (endovaskuläre Hypothermie) eingesetzt. Im Prinzip ist auch denkbar, die Vorrichtung zum Erwärmen von Blut zu verwenden (endovaskuläre Hyperthermie). Der Wärmetauscher 10 wird unabhängig von der Zufuhreinrichtung bzw. dem Zufuhrsystem 21 offenbart und beansprucht. Es ist möglich, den Wärmetauscher 10 gesondert von der Zufuhreinrichtung 21 zur Verfügung zu stellen und beide Komponenten erst während einer Operation zu einem Gesamtsystem zu kombinieren. Zusätzlich wird das System, d.h. die Kombination aus Wärmetauscher 10 und Zufuhreinrichtung 21 offenbart und beansprucht.

Eine Abwandlung des Wärmetauschers 10 gemäß Fig. 5 ist in Fig. 6 gezeigt. Diese unterscheidet sich darin, dass der Dichtungsabschnitt 24 durch eine weitere Versorgungsleitung 28 ersetzt ist, die sich bis zum proximalen Ende des Zufuhrsystems 21 (nicht dargestellt) erstreckt.

In Figur 6 sind die Anschlussstücke 22a, 22b deutlich erkennbar. Die Anschlussstücke 22a, 22b können ein Metall aufweisen bzw. aus einem Metall bestehen. Das Metall umfasst oder ist vorzugsweise eine Nickel-Titan-Legierung , insbesondere Nitinol. Ausnehmungen zur Aufnahme der Röhrchen 23 können beispielsweise durch Laserschneiden gebildet werden. Die Röhrchen 23 sind in den Ausnehmungen angeordnet und mit diesen vorzugweise stoffschlüssig verbunden, beispielsweise durch Schweissen.

Die Anschlussstücke 22a, 22b können auch ein Harz umfassen oder aus einem Harz bestehen. Die Herstellung der Vorrichtung kann dabei einen Schritt umfassen, wobei die zu einem Bündel zusammengefassten Röhrchen 23 an beiden Enden in ein Harz getaucht werden. Vorzugsweise erfolgt dies in einer Zentrifuge, so dass eine gute und homogene Verteilung des Harzes sichergestellt ist. Nach dem Aushärten des Harzes sind die Röhrchen 23 im jeweiligen Anschlussstück 22a, 22b fixiert. Das beschriebene Verfahren erlaubt es, eine vergleichsweise hohe Anzahl von Röhrchen 23 pro Anschlussstück 22a, 22b bereitzustellen. Zudem zeichnet sich ein derartiges Herstellungsverfahren durch eine verbesserte Wirtschaftlichkeit aus.

Die Vorrichtung ist vorzugsweise zur Kombination mit Zufuhrsystemen 21 unterschiedlicher Größen geeignet ist. Insbesondere kann die Vorrichtung so ausgelegt sein, dass sie durch Zufuhreinrichtungen, insbesondere Katheter, unterschiedlicher Größen schiebbar ist. Beispielsweise kann die Vorrichtung im komprimierten Zustand einen Außendurchmesser aufweisen, der ausreichend klein ist, so dass die Vorrichtung in eine Zufuhreinrichtung 21, konkret einen Katheter, einschiebbar ist, der eine Größe zwischen 2 Fr und 9 Fr, insbesondere zwischen 3 Fr und 4 Fr oder zwischen 6 Fr und 9 Fr, aufweist. Auf diese Weise kann die Vorrichtung unabhängig vom bereits gelegten Katheter eingesetzt werden. Der Dichtungsabschnitt 24 kann so ausgelegt bzw. dimensioniert sein, dass die Dichtungsfunktion in Zufuhreinrichtungen 21 unterschiedlicher Größe erfüllt ist. So kann der Dichtungsabschnitt 24 Elemente aufweisen, die sich radial komprimieren lassen.

**Bezugszeichenliste**

| | |
|---|---|
| 10 | Wärmetauscher |
| 11 | Führungsmittel |
| 11a, 11b | Hohlfasern |
| 12 | Wand des Führungsmittels |
| 14 | Wandung des Wärmetauschers |
| 15 | Gitterstruktur |
| 16 | Zwischenlage |
| 17 | Einzelsegmente |
| 19 | Zufuhrleitung |
| 20 | Lumen |
| 21 | Zufuhrsystem |
| 22a | proximales Anschlussstück |
| 22b | distales Anschlussstück |
| 23 | Röhrchen |
| 24 | Dichtungsabschnitt |
| 25 | Mündungsöffnung |
| 26 | Verteiler |
| 27 | Kappe |
| 28 | weitere Versorgungsleitung |

## Patentansprüche

1. Medizinische Vorrichtung zum endovaskulären Kühlen oder Erwärmen von Blut mit einem komprimierbaren und expandierbaren Wärmetauscher (10), der in ein Blutgefäß einführbar ist und wenigstens ein Führungsmittel (11) aufweist, dessen Lumen mit einem Kühl- oder Heizmedium durchströmbar ist und eine Wand (12) aufweist, deren Innenseite zumindest teilweise das Lumen des Führungsmittels (11) begrenzt, deren Außenseite im Gebrauch zumindest teilweise mit dem Blut in Berührung kommt und die für den Wärmeübergang zwischen dem Medium und dem Blut angepasst ist,
**dadurch gekennzeichnet, dass**
der Wärmetauscher (10) eine selbstexpandierbare Wandung (14) aufweist, die im expandierten Zustand die Außenkontur des Wärmetauschers (10) bildet, wobei das Führungsmittel (11) zumindest abschnittsweise die Wandung (14) bildet oder zumindest abschnittsweise mit der Wandung (14) verbunden ist derart, dass das Führungsmittel (11) beim Expandieren der Wandung (14) mitbewegt wird.

2. Vorrichtung nach Anspruch 1,
**dadurch gekennzeichnet, dass**
das Führungsmittel (11) wenigstens eine flexible Hohlfaser (11a, 11b) bildet.

3. Vorrichtung nach Anspruch 1 oder 2,
**dadurch gekennzeichnet, dass**
die Wandung (14) eine Gitterstruktur (15), insbesondere eine geflochtene oder lasergeschnittene Gitterstruktur (15) aufweist.

4. Vorrichtung nach Anspruch 3,
**dadurch gekennzeichnet, dass**
die Hohlfaser (11a, 11b) teilweise oder vollständig die geflochtene Gitterstruktur der Wandung (14) bilden.

5. Vorrichtung nach Anspruch 3,
**dadurch gekennzeichnet, dass**
die Hohlfaser (11a, 11b) in die Gitterstruktur (15) eingeflochten oder mit der Gitterstruktur (15) fest, insbesondere stoffschlüssig, verbunden ist.

6. Vorrichtung nach Anspruch 3,
**dadurch gekennzeichnet, dass**
die Hohlfaser (11a, 11b) eine zusätzliche Gitterstruktur bildet, die mit der Wandung (14) fest verbunden ist.

7. Vorrichtung nach einem der vorhergehenden Ansprüche,
**dadurch gekennzeichnet, dass**
die Wand des Führungsmittels (11) fluiddicht oder porös ist.

8. Vorrichtung nach einem der vorhergehenden Ansprüche,
**dadurch gekennzeichnet, dass**
der Wärmetauscher (10) wenigstens eine Zwischenlage (16) aufweist, die sich in Richtung der Blutströmung erstreckt und wenigstens ein Führungsmittel (11) für das Kühl- oder Heizmedium aufweist.

9. Vorrichtung nach einem der Ansprüche 3 bis 8,
**dadurch gekennzeichnet, dass**
die Wandung (14) wenigstens zwei gliederartige Einzelsegmente (17) aufweist, die nachfolgend angeordnet und unabhängig voneinander komprimierbar und expandierbar sind.

10. Vorrichtung nach einem der vorhergehenden Ansprüche,
**dadurch gekennzeichnet, dass**
das Führungsmittel (11) für die Zufuhr des Mediums mit einem Einlass und für die Abfuhr des Mediums mit einem Auslass zur Verbindung mit einer Versorgungseinrichtung für das Medium verbunden sind.

11. Vorrichtung nach einem der vorhergehenden Ansprüche,
**dadurch gekennzeichnet, dass**
der Wärmetauscher ein Reservoir aufweist, das mit dem Führungsmittel (11) verbunden und im Gebrauch mit dem Medium füllbar und entleerbar ist.

12. Vorrichtung nach einem der vorhergehenden Ansprüche,
**dadurch gekennzeichnet, dass**
der mehrere Führungsmittel in gesonderten Modulen angeordnet sind, deren Fläche jeweils kleiner als die Fläche der gesamten Wandung des Wärmetauschers ist.

13. Vorrichtung nach einem der Anspruch 12,
**dadurch gekennzeichnet, dass**
die Module unabhängig voneinander mit dem Medium versorgbar sind oder strömungstechnisch parallel geschaltet sind.

14. Vorrichtung nach einem der Ansprüche 1 bis 13, **dadurch gekennzeichnet, dass** das Temperiermodul (13) einen Expansionskörper aus mehreren Einzelröhrchen 23 aufweist.

15. Medizinisches System mit einer Vorrichtung nach einem der vorhergehenden Ansprüche und einer Zufuhreinrichtung, insbesondere einer Schleuse, wobei die Vorrichtung längsverschieblich in der Schleuse angeordnet ist.
